(19) Europäisches Patentamt
European Patent Office
Office européen des brevets

(11) Veröffentlichungsnummer: **0 189 370**
**A2**

(12) # EUROPÄISCHE PATENTANMELDUNG

(21) Anmeldenummer: **86810003.3**

(22) Anmeldetag: **08.01.86**

(51) Int. Cl.⁴: **C 07 D 491/10**, C 07 D 497/10, C 07 D 495/10, A 61 K 31/435, A 61 K 31/40 // C07D211/48, C07D211/54, (C07D491/10, 317:00, 221:00), (C07D491/10, 317:00, 209:00), (C07D497/10, 327:00, 221:00), (C07D495/10, 327:00, 209:00), (C07D495/10, 339:00, 221:00), (C07D495/10, 339:00, 209:00)

(30) Priorität: **16.01.85 DE 3501225**
**08.02.85 DE 3504286**
**08.02.85 DE 3504284**
**08.02.85 DE 3504281**

(43) Veröffentlichungstag der Anmeldung: **30.07.86 Patentblatt 86/31**

(84) Benannte Vertragsstaaten: **AT BE CH DE FR GB IT LI LU NL SE**

(71) Anmelder: **SANDOZ AG, Lichtstrasse 35, CH-4002 Basel (CH)**
(84) Benannte Vertragsstaaten: **BE CH FR GB IT LI LU NL SE**

(71) Anmelder: **SANDOZ-PATENT-GMBH, Humboldtstrasse 3, D-7850 Lörrach (DE)**
(84) Benannte Vertragsstaaten: **DE**

(71) Anmelder: **SANDOZ-ERFINDUNGEN Verwaltungsgesellschaft m.b.H., Brunner Strasse 59, A-1235 Wien (AT)**
(84) Benannte Vertragsstaaten: **AT**

(72) Erfinder: **Bolliger, Georg, Benkenstrasse 43, CH-4102 Binningen (CH)**

(54) **Spiro-dioxolane, -dithiolane und -oxothiolane.**

(57) Spiro-Verbindungen, enthaltend einen fünfgliedrigen, carbocyclischen Ring, der in den Stellungen 1 und 3 durch zwei Heteroatome aus der Reihe Sauerstoff und Schwefel unterbrochen ist und dessen Kohlenstoffatom in Stellung 4 mit einem 4- bis 8-gliedrigen, carbocyclischen Ring, der durch ein Stickstoffatom unterbrochen ist, gemeinsam ist, in Form der freien Basen oder von pharmazeutisch annehmbaren Säureadditionssalzen oder quaternären Ammonium-Salzen, ihre Herstellung und Anwendung als Therapeutika.

EP 0 189 370 A2

0189370

SANDOZ AG
4002 Basel

Case 100-6554

# SPIRO-DIOXOLANE, -DITHIOLANE UND -OXOTHIOLANE

Gegenstand der vorliegenden Erfindung ist die Anwendung bei der therapeutischen Behandlung von Spiro-dioxolanen, -dithiolanen und -oxothiolanen.

Die Erfindung umfasst insbesondere Spiro-Verbindungen, enthaltend einen fünfgliedrigen, carbocyclischen Ring, der in den Stellungen 1 und 3 durch zwei Heteroatome aus der Reihe Sauerstoff und Schwefel unterbrochen ist und dessen Kohlenstoffatom in Stellung 4 mit einem 4- bis 8-gliedrigen, carbocyclischen Ring, der durch ein Stickstoffatom unterbrochen ist, gemeinsam ist, in Form der freien Basen oder von pharmazeutisch annehmbaren Säureadditionssalzen oder quaternären Ammonium-Salzen, zur Anwendung als Therapeutika.

Diese im folgenden als erfindungsgemässe Verbindungen bezeichneten Spiro-Verbindungen weisen folgendes Grundgerüst auf,

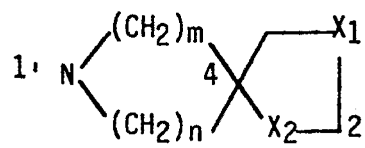

worin $X_1$ und $X_2$ unabhängig voneinander Sauerstoff oder Schwefel bedeuten und m und n unabhängig voneinander für 1, 2, 3 oder 4 stehen, wobei m + n nicht mehr als 6 beträgt.

Dieses Grundgerüst kann pharmakologisch verträgliche Gruppen aufweisen, z.B. in den Stellungen 2 und 1'.

Die erfindungsgemässen Verbindungen weisen, wenn m und n verschieden sind, ein asymmetrisches Kohlenstoffatom in Stellung 4 auf, und je nach den vorhandenen Substituenten auch in weiteren Stellungen, z.B. in Stellung 2. Sie können deshalb in racemischer oder optisch aktiver Form vorliegen. Die Erfindung betrifft sowohl die Racemate wie auch die Enantiomere.

Die erfindungsgemässen Verbindungen können in freier Form oder als pharmazeutisch annehmbare Säureadditionssalze oder quaternäre Ammonium-Salze vorliegen. Die Erfindung betrifft sowohl die freie Form wie auch die Salzformen. Beispiele von Säureadditionsformen sind die Hydrochloride, die Hydrogenfumarate und die Hydrogenmaleinate. Beispiele von Ammonium-Salzformen sind die Iodomethylate.

Insbesondere werden Verbindungen der Formel I umfasst,

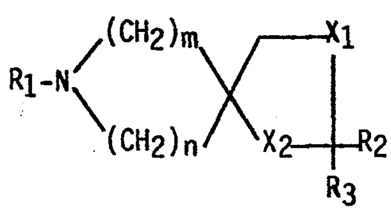

I

worin

$R_1$ für Wasserstoff, gegebenenfalls durch 1 bis 6 Halogenatome mit einer Atomzahl von 9 bis 35 substituiertes Alkyl mit

1 bis 6 Kohlenstoffatomen, Alkenyl oder Alkynyl mit je 3 bis 6 Kohlenstoffatomen, wobei die Mehrfachbindung nicht zu dem Stickstoffatom benachbart ist, Cycloalkyl mit 3 bis 7 Kohlenstoffatomen, Cycloalkyl(mit 3 bis 7 Kohlenstoffatomen)-alkyl(mit 1 oder 2 Kohlenstoffatomen), Benzyl, Alkoxycarbonyl mit 2 bis 5 Kohlenstoffatomen, Benzoxycarbonyl, Alkanoyl mit 2 bis 5 Kohlenstoffatomen, Benzoyl, Nicotinoyl, Dihydronicotinoyl oder N-Alkyl(mit 1 bis 4 Kohlenstoffatomen)-dihydronicotinoyl steht,

$R_2$ und $R_3$ unabhängig voneinander Wasserstoff, gegebenenfalls durch 1 bis 6 Halogenatome mit einer Atomzahl von 9 bis 35 substituiertes Alkyl mit 1 bis 6 Kohlenstoffatomen, Alkenyl oder Alkynyl mit je 2 bis 6 Kohlenstoffatomen, Cycloalkyl mit 3 bis 7 Kohlenstoffatomen oder Phenyl bedeuten oder zusammen eine $-(CH_2)_p$-Kette bilden, wobei p für 2, 3, 4 oder 5 steht,

$X_1$ und $X_2$ unabhängig voneinander Sauerstoff oder Schwefel bedeuten, und

m und n unabhängig voneinander für 1, 2, 3 oder 4 stehen, wobei m + n nicht mehr als 6 beträgt,

in Form der freien Basen oder von pharmazeutisch annehmbaren Säureadditionssalzen und quaternären Ammonium-Salzen.

Sofern in den Verbindungen der Formel I vorstehend definierte Alkyl- oder Alkoxygruppen enthalten sind, besitzen diese vorzugsweise 1 oder 2 Kohlenstoffatome und stellen insbesondere Methyl oder Methoxy dar. Sofern ein Substituent für vorstehend definiertes Halogen steht, stellt er vorzugsweise Fluor oder Chlor dar. Bevorzugt stehen m und n für 2 und 2, 3 und 1 oder 2 und 1.

Man gelangt zu den erfindungsgemässen Verbindungen, indem man eine entsprechende 4- bis 8-gliedrige, durch ein Stickstoffatom unterbrochene, carbocyclische Verbindung, die anstelle des fünfgliedrigen heterocyclischen Ringes eine Gruppe aus der Reihe Hydroxy und Mercapto und eine Gruppe aus der Reihe Hydroxymethyl und Mercaptomethyl aufweist, cyclisiert und die erhaltene Spiro-Verbindung oder eine daraus hergestellte, weitere erfindungsgemässe Verbindung, in Form der freien Base oder eines pharmazeutisch annehmbaren Säureadditionssalzes oder quaternären Ammonium-Salzes gewinnt.

Insbesondere gelangt man zu den Verbindungen der Formel I und ihren pharmazeutisch annehmbaren Säureadditionssalzen und quaternären Ammonium-Salzen über ein Verfahren, enthaltend die Stufe, dass man eine Verbindung der Formel II,

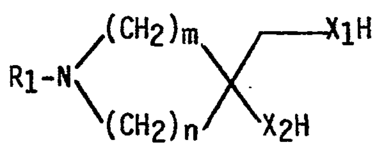

II

worin $R_1$, $X_1$, $X_2$, m und n obige Bedeutung besitzen, zu einer Verbindung der Formel I cyclisiert, und die erhaltene Verbindung der Formel I oder eine daraus hergestellte, weitere Verbindung der Formel I in Form der freien Base oder eines pharmazeutisch annehmbaren Säureadditionssalzes oder quaternären Ammonium-Salzes gewinnt.

Vorzugsweise wird die Verbindung der Formel II mit einer Verbindung der Formel III,

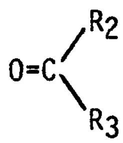

III

worin $R_2$ und $R_3$ obige Bedeutung besitzen, umgesetzt.

Die Umsetzung erfolgt unter wasserabspaltenden Bedingungen und kann in bekannter Weise durchgeführt werden, beispielsweise unter milder Lewissäure-Katalyse, z.B. mittels Bortrifluoridätherat.

Eine so erhaltene Verbindung der Formel I kann nach an sich bekannten Methoden in eine weitere Verbindung der Formel I übergeführt werden.

So kann eine Verbindung der Formel I, worin $R_1$ für Alkoxycarbonyl mit 2 bis 5 Kohlenstoffatomen steht, in bekannter Weise, beispielsweise mittels Lithiumaluminiumhydrid oder Aluminiumhydrid, in eine Verbindung der Formel I, worin $R_1$ für Methyl steht, reduziert werden.

Ferner kann eine Verbindung der Formel I, worin $R_1$ für Wasserstoff steht, nach einer für die Alkylierung oder Acylierung auf einem sekundären Stickstoff bekannten Methode in eine Verbindung der Formel I, worin $R_1$ eine andere Bedeutung als Wasserstoff besitzt, übergeführt werden.

Die Aufarbeitung der gemäss obigen Verfahren erhaltenen Reaktionsgemische und die Reinigung der so gewonnenen Verbindungen der Formel I kann nach an sich bekannten Methoden durchgeführt werden.

Die erhaltenen Racemate können in die individuellen optisch aktiven Komponenten gespalten werden, wobei bekannte Methoden zur Anwendung gelangen, z.B. via vorübergehende Säureadditionssalzbildung mit optisch aktiven Säuren, und fraktionierter Kristallisation der diastereoisomeren Säureadditionssalze.

Aus den Verbindungen der Formel I in freier Form lassen sich in bekannter Weise Säureadditionssalze und quaternäre Ammonium-Salze herstellen und umgekehrt.

Die Ausgangsverbindungen der Formel II sind bekannt oder können wie folgt hergestellt werden:

Die Verbindungen der Formel II, worin $X_1$ und $X_2$ beide für Sauerstoff stehen, werden z.B. erhalten, indem man Verbindungen der Formel IV,

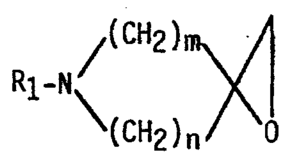

IV

worin $R_1$, m und n obige Bedeutung besitzen, auf an sich bekannte Weise hydrolysiert.

Die Verbindungen der Formel II, worin $X_1$ für Schwefel und $X_2$ für Sauerstoff steht, werden z.B. erhalten, indem man Verbindungen der Formel IV mit Thioessigsäure umsetzt und die intermediär entstandenen Thioacetate basisch hydrolysiert.

Die Verbindungen der Formel II, worin $X_1$ und $X_2$ beide für Schwefel stehen, werden z.B. erhalten, indem man Verbindungen der Formel V,

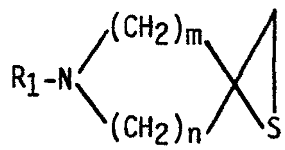

V

worin $R_1$, m und n obige Bedeutung besitzen, mit Thioessigsäure umsetzt und die intermediär entstandenen Thioacetate basisch hydrolysiert.

Die Verbindungen der Formel II, worin $X_1$ für Sauerstoff und $X_2$ für Schwefel steht, werden z.B. erhalten, indem man Verbindungen der Formel V mit Essigsäureanhydrid umsetzt und die intermediär entstandenen Acetate basisch oder sauer hydrolysiert.

Die Verbindungen der Formel V können auf an sich bekannte Weise aus Verbindungen der Formel IV hergestellt werden, z.B. durch Umsetzung mit Thioharnstoff.

Die Verbindungen der Formel IV können z.B. erhalten werden, indem man Verbindungen der Formel VI,

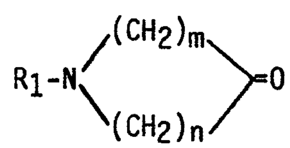

VI

worin $R_1$, m und n obige Bedeutung besitzen, mit Dimethylsulfoxonium-methylid umsetzt.

Soweit die Herstellung der Ausgangsprodukte nicht beschrieben wird, sind diese bekannt oder nach an sich bekannten Verfahren bzw. analog zu an sich bekannten Verfahren herstellbar.

Die Verbindungen der Formel I, worin $X_1$ und $X_2$ beide für Sauerstoff stehen, können ebenfalls erhalten werden, indem man die Verbindungen der Formel IV direkt mit den Verbindungen der Formel III, beispielsweise unter milder Lewissäure-Katalyse umsetzt, z.B. wie im Beispiel 2 erläutert.

Von den erfindungsgemässen Verbindungen sind das 3,7-Dimethyl-2,4-dioxo-7-azaspiro[3,4]octan und dessen Iodomethylat, das [2,2-Dimethyl-spiro-(1,3-dioxolan-4,3')]-1'-methyl-piperidin-2-on, das Spiro-(1,3-dioxolan-4,4')-1'-t.-butyl-piperidin und das [2,2-Dimethyl-spiro-(1,3-dioxolan-4,4')]-1'-t.-butyl-piperidin aus der Literatur bekannt, z.B. aus H.F. Ridley et al., J. Med. Chem. 1969, Vol. 12, S. 931 - 933, M.L. Rueppel et al., J. Amer. Chem. Soc. 1972, Vol. 94, Nr. 11, S. 3877 - 3883 und R.A.Y. Jones et al., J. Chem. Soc. Perkin Trans. 2, 1973, Nr. 4, S. 337 - 338. Es wurde jedoch bis jetzt für diese Verbindungen keine pharmazeutische Wirkung beschrieben.

Es wurde nun überraschenderweise gefunden, dass die erfindungsgemässen Verbindungen im Tierversuch interessante pharmakodynamische Eigenschaften aufweisen und daher als Heilmittel verwendet werden können.

Die erfindungsgemässen Verbindungen bewirken im Beobachtungstest bei der Maus, mit Dosen von 1 bis 300 mg/kg p.o., eine Verlängerung des Wachzustandes sowie eine erhöhte Reaktivität auf externe Reize.

Im Schlaf-Wach-Zyklus bei der chronisch implantierten Ratte bewirken sie mit 1 bis 100 mg/kg p.o. eine Erhöhung des REM-Schlafes.

Ferner verstärken sie bei 1 bis 300 mg/kg p.o. die $^{14}C$-Deoxy-glucose-Aufnahme in bestimmten Gehirnarealen, insbesondere im vorderen Thalamus (Methode nach L. Sokoloff, Journal of Cerebral Blood Flow and Metabolism 1981, 1, 7 - 36, H.E. Savaki et al., Brain Research 1982, 233, 347 und J. McCulloch et al., Journal of Cerebral Blood Flow and Metabolism 1981, 1, 133 - 136).

Aufgrund dieses muskarinisch cholinergen Profils können sie für die Indikationen senile Demenz, Alzheimer'sche Krankheit, Huntingtons chorea, tardive Diskinesia, Hyperkinesia, akute Verwirrtheitszustände und Manie, ferner zur Behandlung des Glaukoms verwendet werden.

Eine geeignete Tagesdosis liegt im Bereich von etwa 1 bis 100 mg der Substanz, geeignete Dosierungsformen für z.B. orale Anwendungen enthalten im allgemeinen ungefähr 0,3 bis 50 mg wirksame Substanz neben festen oder flüssigen Trägersubstanzen oder Verdünnungsmitteln.

Als Heilmittel können die erfindungsgemässen Verbindungen allein oder in geeigneter Arzneiform mit pharmakologisch indifferenten Stoffen verabreicht werden.

Gegenstand der Erfindung sind auch pharmazeutische Zusammensetzungen, die die erfindungsgemässen Verbindungen als Wirkstoffe enthalten. Für ihre Herstellung können die in der Pharmazie gebräuchlichen Hilfs- und Trägerstoffe verwendet werden. Geeignete galenische Formen sind z.B. Tabletten, Kapseln und Tropflösungen. Diese pharmazeutischen Zusammensetzungen können gegebenenfalls einen peripheren cholinergen Blocker enthalten.

Solche Blocker sind bekannt und umfassen z.B. Methylscopolamin, Methylatropin, Tropenzilium und Pirenzepin.

Die erfindungsgemässen Verbindungen mit Ausnahme der oben erwähnten, aus der Literatur vorbekannten Verbindungen, sind neu und ebenfalls Gegenstand der vorliegenden Erfindung, sowie ihr oben beschriebenes Herstellungsverfahren.

Diese Verbindungen umfassen insbesondere die Verbindungen der Formel I, worin $X_1$ und $X_2$ nicht beide Sauerstoff bedeuten, falls

a) $R_1$ und $R_2$ Methyl bedeuten, $R_3$ für Wasserstoff steht und m und n beide für 1 stehen, oder

b) $R_1$ t.-Butyl bedeutet, $R_2$ und $R_3$ beide für Wasserstoff oder beide für Methyl und m und n beide für 2 stehen,

und ihre pharmazeutisch annehmbaren Säureadditionssalze und quaternären Ammonium-Salze.

Die Erfindung umfasst z.B. die oben definierten, neuen Verbindungen der Formel I, als freie Basen oder als Säureadditionssalze.

Die nachfolgenden Beispiele erläutern die Erfindung. Temperaturangaben erfolgen in Celsiusgraden und sind unkorrigiert.

Beispiel 1: [2-Methyl-spiro-(1,3-dioxolan-4,4')]-1'-äthoxycarbonyl-piperidin

8 g 1-Aethoxycarbonyl-4-hydroxy-4-hydroxymethyl-piperidin in 100 ml Toluol werden auf - 10 ° abgekühlt und mit 40 ml Acetaldehyd versetzt. Nun werden bei gleichbleibender Temperatur langsam 20 ml Bortrifluorid-ätherat zugetropft. Man rührt noch 2 Stunden nach und arbeitet dann wie folgt auf:

Die braune Suspension wird mit 200 ml 2 N Natronlauge-Lösung basisch gestellt und dreimal mit 300 ml Methylenchlorid extrahiert. Die vereinigten organischen Phasen werden mit Wasser gewaschen, über Natriumsulfat getrocknet, filtriert und eingedampft. Der braune ölige Rückstand wird am Vakuum destilliert. Man erhält 6,5 g farbloses Oel.

Sdp.: 124 - 127 °/0,08 mm Hg.

Das als Ausgangsmaterial benötigte 1-Aethoxycarbonyl-4-hydroxy-4-hydroxymethyl-piperidin kann wie folgt hergestellt werden:

a) Spiro-(oxiran-2,4')-1'-äthoxycarbonyl-piperidin

Dimethylsulfoxonium-methylid wird nach Corey et al., Organic Synth. 49, 78 (1969) aus 17,5 g Natriumhydrid, 70 g Trimethylsulfoxonium-jodid in 500 ml Dimethylsulfoxid in einem 1,5 Liter Sulfierkolben mit einem mechanischen Rührer, Rückflusskühler und Gaseinleitungsrohr unter Feuchtigkeitsschutz hergestellt. Nach vollständiger Reaktion wird das Gaseinleitungsrohr durch einen Tropftrichter mit Druckausgleich ersetzt, der 50 g N-Carbäthoxy-4-piperidon in 150 ml trockenem Dimethylsulfoxid enthält. Diese Lösung wird während

15 Minuten zum vorgelegten Dimethylsulfoxonium-methylid zugetropft. Man lässt noch 1 Stunde weiterrühren und arbeitet dann die gelbe Lösung wie folgt auf:

Die gelbe Lösung wird unter gutem Rühren auf 1,2 Liter Eis gegossen und mit 1,5 Liter Aether extrahiert. Nach zweimaligem Nachextrahieren mit je 1,5 Liter Aether und Waschen mit 600 ml Sole/Wasser (1 : 1) werden die Aetherphasen vereinigt, über Natriumsulfat getrocknet, filtriert und eingedampft. Das zurückbleibende gelbe Oel wird am Vakuum destilliert.

Man erhält 32 g farbloses, bewegliches Oel.

Sdp.: 90 - 92 °/0,1 mm Hg.

b) 1-Aethoxycarbonyl-4-hydroxy-4-hydroxymethyl-piperidin

8,3 g Spiro-(oxiran-2,4')-1'-äthoxycarbonyl-piperidin werden in 200 ml 0,02 N Salzsäurelösung hydrolysiert, indem man 1 Stunde auf 50 ° erhitzt. Nach Abkühlen wird die Lösung mit 2 N Sodalösung neutralisiert und dreimal mit 500 ml Methylenchlorid extrahiert. Die vereinigten organischen Phasen werden mit gesättigter Kochsalzlösung gewaschen, über Natriumsulfat getrocknet, filtriert und eingedampft. Man erhält 8,1 g des gewünschten Diols als reines farbloses Harz, das ohne weitere Reinigung für die Cyclisationsreaktion eingesetzt wird.

Beispiel 2: [2-Methyl-spiro-(1,3-dioxolan-4,4')]-1'-äthoxy-carbonyl-piperidin

26 g Spiro-(oxiran-2,4')-1'-äthoxycarbonyl-piperidin [Herstellung siehe Beispiel 1 a)] in 200 ml Toluol werden auf - 10 ° abgekühlt und mit 80 ml Acetaldehyd versetzt. Nun werden bei gleichbleibender Temperatur langsam 35 ml Bortrifluorid-ätherat zugetropft. Man rührt noch 2 Stunden nach und arbeitet dann wie folgt auf:

Die braune Suspension wird mit 300 ml 2 N Natronlauge-Lösung basisch gestellt und dreimal mit 500 ml Methylenchlorid extrahiert. Die vereinigten organischen Phasen werden mit Wasser gewaschen, über Natriumsulfat getrocknet, filtriert und eingedampft. Der braune ölige Rückstand wird am Vakuum destilliert. Man erhält 21 g farbloses Oel.

Sdp.: 124 - 127 °/0,08 mm Hg.

Analog zu Beispiel 1 oder 2 werden folgende Verbindungen hergestellt, worin $X_1$ und $X_2$ je für Sauerstoff stehen (alles Racemate, gegebenenfalls Gemische von cis- und trans-Diastereomeren):

| Beispiel | $R_1$ | $R_2$ | $R_3$ | m | n | Smp. |
|---|---|---|---|---|---|---|
| 3 | 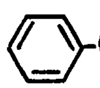-$CH_2$- | -$CH_3$ | H | 2 | 2 | 176 - 179 ° 1, 5 |
| 4 | H | -$CH_3$ | H | 2 | 2 | 157 - 160 ° 1, 6 |
| 5 | $H_3C$- | -$CH_3$ | H | 2 | 2 | 161 - 164 ° 2, 7 |
| 6 | 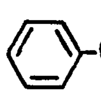-$CH_2$- | -$CH_3$ | H | 3 | 1 | 186 - 188 ° 1, 5 |

| Beispiel | $R_1$ | $R_2$ | $R_3$ | m | n | Smp. |
|---|---|---|---|---|---|---|
| 7 | H | $-CH_3$ | H | 3 | 1 | 144 - 146 ° [2, 7] |
| 8 | $C_2H_5$-O-CO- | $-CH_3$ | H | 3 | 1 | farbloses Oel [10] |
| 9 | $H_3C$- | $-CH_3$ | H | 3 | 1 | 159 - 162 ° [3, 6] |
| 10 | $C_2H_5$-O-CO- | $-C_2H_5$ | H | 2 | 2 | farbloses Oel [11] |
| 11 | $H_3C$- | $-C_2H_5$ | H | 2 | 2 | 110 - 113 ° [4, 8] |
| 12 | $H_3C$- | 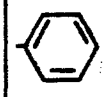 | H | 2 | 2 | 165 - 168 ° [4, 9] |
| 13 | $H_3C$- | $-CH_3$ | $-CH_3$ | 2 | 2 | 141 - 143 ° [4, 8] |
| 14 | $H_3C$- | $-CH_3$ | H | 2 | 1 | 87 - 89 ° [3, 6] |
| 15 | $H_3C$- | -C≡CH | H | 2 | 2 | 117-120° [4,8] |
| 16 | $H_3C$- | 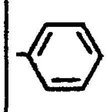 | 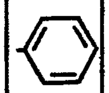 | 2 | 2 | 175-178° [4,8] |
| 17 | $H_3C$- | H | H | 2 | 2 | 119-122° [4,8] |
| 18 | $H_3C$- | $-CCl_3$ | H | 2 | 2 | 151-154° [4,5] |
| 19 | $H_3C$- | $-(CH_2)_3-$ | | 2 | 2 | 153-155° [4,5] |

1) Hydrochlorid
2) Hydrogenfumarat
3) Hydrogenoxalat
4) Hydrogenmaleinat
5) aus Methylenchlorid/Aether
6) aus Aceton/Essigester
7) aus Aceton/Aether
8) aus Aethanol/Aether
9) aus Isopropanol/Aether
10) Sdp.: 98 - 102 °/0,05 mm Hg
11) Sdp.: 148 - 150 °/ 0,3 mm Hg

Das Iodomethylat des im Beispiel 5 erhaltenen racemischen [2-Methyl-spiro-(1,3-dioxolan-4,4')]-1'-methyl-piperidin wird hergestellt, indem man zu einer Lösung der freien Base in Aceton ein Ueberschuss von Methyljodid zugibt und die ausgefallenen Kristalle nach 12 Stunden abnutscht. Smp.: 226 - 228 ° C (aus Aceton).

Beispiel 20: (+)- und (-)-[2-Methyl-spiro-(1,3-dioxolan-4,4')]-1'-methyl-piperidin

Das im Beispiel 5 erhaltene racemische [2-Methyl-spiro-(1,3-dioxolan-4,4')]-1'-methyl-piperidin (freie Base) wird mit (-)-Di-O,O'-p-toluyl-L-Weinsäure resp. (+)-Di-O,O'-p-toluyl-D-Weinsäure und fraktionierte Kristallisation in die beiden Enantiomeren aufgetrennt:

| Beispiel 20a: (-)-Enantiomeres | Beispiel 20b: (+)-Enantiomeres |
|---|---|
| Sdp.: 92 - 95 °/10 mm Hg<br>$[\alpha]_D^{20}$ = - 23,7 ° (c = 2,0 in Aethanol)<br>Hydrogenmaleinat aus Isopropanol/Aether:<br>Smp.: 136 - 138 °<br>$[\alpha]_D^{20}$ = - 12,9 ° (c = 0,62 in Aethanol)<br>Iodomethylat aus Aceton:<br>Smp.: 234 - 236 °<br>$[\alpha]_D^{20}$ = - 11,1 ° (c = 0,5 in Aethanol) | Sdp.: 92 - 95 °/10 mm Hg<br>$[\alpha]_D^{20}$ = + 23,6 ° (c = 1,1 in Aethanol)<br>Hydrogenmaleinat aus Isopropanol/Aether:<br>Smp.: 136 - 138 °<br>$[\alpha]_D^{20}$ = + 12,9 ° (C = 1,2 in Aethanol)<br>Iodomethylat aus Aceton:<br>Smp.: 234 - 236 °<br>$[\alpha]_D^{20}$ = + 11,0 ° (c = 0,6 in Aethanol) |

Beispiel 21: cis- und trans-[2-Methyl-spiro-(1,3-dioxolan-4,3')]-1'-methyl-pyrrolidin

Das im Beispiel 14 erhaltene cis/trans-Diastereomerengemisch von [2-Methyl-spiro-(1,3-dioxolan-4,3')]-1'-methyl-pyrrolidin wird chromatographisch in die cis-Diastereomeren (Racemat) und die trans-Diastereomeren aufgetrennt:

| Beisp. 21a: cis-Diastereomeren | Beisp. 21b: trans-Diastereomeren |
|---|---|
| Hydrogenmaleinat aus Methylenchlorid/Aether: Smp.: 78 - 81 ° | Hydrogenmaleinat aus Methylenchlorid/Aether: Smp.: 94 - 97 ° |

Beispiel 22: [2-Methyl-spiro-(3-oxo-1-thiolan-4,4')]-1'-äthoxycarbonyl-piperidin

a) 1-Aethoxycarbonyl-4-hydroxy-4-mercaptomethyl-piperidin

10 g Spiro-(oxiran-2,4')-1'-äthoxycarbonyl-piperidin [Herstellung siehe Beispiel 1 a)] werden unter Stickstoff zu 40 ml auf 0 ° gekühlte Thioessigsäure zugetropft. Nach 3 Stunden Rühren bei 0 ° wird mit 2 N Sodalösung basisch gestellt und dreimal mit 400 ml Methylenchlorid extrahiert. Die organischen Phasen werden vereinigt, über Natriumsulfat getrocknet, filtriert und eingedampft. Das klare, gelbe Oel (15,3 g) wird ohne weitere Reinigung in 100 ml Methanol aufgenommen und mit 10 g fester Potasche versetzt. Nach 6 Stunden Rühren bei Zimmertemperatur unter Stickstoff wird die Suspension über Hyflo filtriert und das Filtrat zwischen

200 ml Sole/Wasser (1 : 1) und 600 ml Methylenchlorid verteilt. Nach zweimaligem Nachextrahieren mit je 500 ml Methylenchlorid werden die organischen Phasen vereinigt, über Natriumsulfat getrocknet und eingedampft. Man erhält 12,4 g der Titelverbindung als gelbes Harz, das ohne weitere Reinigung für die Cyclisierung eingesetzt wird.

b) [2-Methyl-spiro-(3-oxo-1-thiolan-4,4')]-1'-äthoxycarbonyl-piperidin

Das unter a erhaltene gelbe Harz wird in 100 ml Toluol aufgenommen, auf - 10 ° gekühlt und mit 40 ml Acetaldehyd versetzt. Nun werden bei gleichbleibender Temperatur langsam 15 ml Bortrifluorid-ätherat zugetropft. Man rührt noch 2 Stunden nach und arbeitet dann wie folgt auf:

Die rotbraune Suspension wird mit 150 ml 2 N Natronlauge-Lösung basisch gestellt und dreimal mit 300 ml Methylenchlorid extrahiert. Die vereinigten organischen Phasen werden mit Wasser gewaschen, über Natriumsulfat getrocknet, filtriert und eingedampft. Der rotbraune ölige Rückstand wird am Vakuum destilliert. Man erhält 4 g gelbliches Oel. Sdp.: 137 - 143 °/0,1 mm Hg.

Beispiel 23: [2-Methyl-spiro-(3-oxo-1-thiolan-4,4')]-1'-methyl-piperidin

Diese Verbindung wird analog zu Beispiel 22 hergestellt. Hydrochlorid aus Methylenchlorid/Aether: Smp.: 138 - 140 °.

Beispiel 24: cis- und trans-[2-Methyl-spiro-(3-oxo-1-thiolan-4,3')]-1'-methyl-pyrrolidin

Das Isomerengemisch wird analog zu Beispiel 17 hergestellt und chromatographisch in die cis-Diastereomeren und die trans-Diastereomeren (jeweils Racemate) aufgetrennt:

| Beisp. 24a: cis-Diastereomeren | Beisp. 24b: trans-Diastereomeren |
|---|---|
| Hydrogenmaleinat aus Methylenchlorid/Aether: Smp.: 106 - 109 ° | Hydrogenmaleinat aus Methylenchlorid/Aether: Smp.: 60 - 65 ° (Zers.) |

Beispiel 25: [2-Methyl-spiro-(1,3-dithiolan-4,4')]-1'-äthoxycarbonyl-piperidin

a) Spiro-(thiiran-2,4')-1'-äthoxycarbonyl-piperidin

Zu einer Suspension von 7,6 g Thioharnstoff in 38,2 ml 15%-ige Schwefelsäure werden bei - 5 ° 18,5 g Spiro-(oxiran-2,4')-1'-äthoxycarbonyl-piperidin [Herstellung siehe Beispiel 1 a)] während 15 Minuten zugetropft. Man rührt noch 2 Stunden weiter und arbeitet die nun entstandene homogene, orange Lösung wie folgt auf:

Man lässt auf Raumtemperatur erwärmen, versetzt die orange Lösung mit 100 ml 2 N Sodalösung, extrahiert viermal mit 400 ml Aether und wäscht die Aetherphasen mit 100 ml Sole. Die organischen Phasen werden vereinigt, über Natriumsulfat getrocknet, filtriert und eingedampft. Der hellgelbe klare

Rückstand wird am Vakuum destilliert. Man erhält 15,6 g der Titelverbindung. Sdp.: 91 - 93 °/0,03 mm Hg.

b) 1-Aethoxycarbonyl-4-mercapto-4-mercaptomethyl-piperidin

Zu einer auf 0 ° gekühlten Lösung von 42 ml Thioessigsäure und 6 g p-Dimethylaminopyridin werden unter Stickstoff 10 g Spiro-(thiiran-2,4')-1'-äthoxycarbonyl-piperidin zugetropft. Nach 2 Stunden Rühren bei 0 ° wird mit 2 N Sodalösung basisch gestellt und dreimal mit 400 ml Methylenchlorid extrahiert. Anschliessend werden die organischen Phasen der Reihe nach mit je 100 ml Wasser, 2 N Salzsäure-Lösung und wieder Wasser gewaschen, vereinigt, über Natriumsulfat getrocknet, filtriert und eingedampft. Man erhält 16,1 g eines gelben, klaren Oels, das ohne weitere Reinigung in 100 ml Methanol aufgenommen und mit 9,3 g fester Potasche versetzt wird. Nach 3 Stunden Rühren unter Stickstoff, wird die Suspension über Hyflo filtriert und das Filtrat zwischen 200 ml Sole/Wasser (1 : 1) und 600 ml Methylenchlorid verteilt. Nach zweimaligem Nachextrahieren mit je 500 ml Methylenchlorid werden die organischen Phasen vereinigt, über Natriumsulfat getrocknet und eingedampft. Man erhält 14,0 g der Titelverbindung als gelbes Harz, das ohne weitere Reinigung für die Cyclisierung eingesetzt wird.

c) [2-Methyl-spiro-(1,3-dithiolan-4,4')]-1'-äthoxycarbonyl-piperidin

Das unter b erhaltene Harz wird in 120 ml Toluol aufgenommen, auf - 10 ° gekühlt und mit 36 ml Acetaldehyd versetzt. Nun werden bei gleichbleibender Temperatur langsam 15 ml Bortrifluorid-ätherat zugetropft. Man rührt noch 2 Stunden nach und arbeitet dann wir folgt auf:

Die rotbraune Suspension wird mit 150 ml 2 N Natronlauge-Lösung basisch gestellt und dreimal mit 300 ml Methylenchlorid extrahiert. Die vereinigten organischen Phasen werden mit Wasser gewaschen, über Natriumsulfat getrocknet, filtriert und eingedampft. Der rotbraune, ölige Rückstand wird am Vakuum destilliert. Man erhält 4,3 g gelbliches Oel. Sdp.: 132 - 138 °/0,02 mm Hg.

Beispiel 26: [2-Methyl-spiro-(1,3-dithiolan-4,4')]-1'-methyl-piperidin

Diese Verbindung wird analog zu Beispiel 25 hergestellt. Hydrogenmaleinat aus Aethanol/Aether: Smp.: 137 - 140 °.

Beispiel 27: [2-Methyl-spiro-(1,3-dithiolan-4,3')]-1'-methyl-pyrrolidin

Das cis/trans-Diastereomerengemisch wird analog zu Beispiel 25 hergestellt. Hydrogenmaleinat aus Methanol/Aether: Smp.: 115 - 119 °.

Beispiel 28: [2-Methyl-spiro-(3-oxo-1-thiolan-5,4')]-1'-äthoxycarbonyl-piperidin

a) 1-Aethoxycarbonyl-4-hydroxymethyl-4-mercapto-piperidin

10 g Spiro-(thiiran-2,4')-1'-äthoxycarbonyl-piperidin [Herstellung siehe Beispiel 25 a)] werden in 5 ml Essigsäureanhydrid und 0,4 ml Pyridin gelöst und während 24 Stunden auf 100 ° erhitzt. Nach Abkühlen auf Zimmertemperatur wird mit 2 N Sodalösung basisch gestellt und dreimal mit 200 ml Methylenchlorid extrahiert. Anschliessend werden die organischen Phasen der Reihe nach mit je 50 ml Wasser, 2 N Salzsäurelösung und wieder mit Wasser gewaschen, vereinigt, über Natriumsulfat getrocknet, filtriert und eingedampft. Man erhält 15,8 g eines gelben klaren Oels, das ohne weitere Reinigung in 100 ml Methanol, das 5 % HCl-Gas enthält, aufgenommen wird. Nach 16 Stunden Rühren wird zwischen 200 ml Sole/Wasser (1 : 1) und 600 ml Methylenchlorid verteilt. Nach zweimaligem Nachextrahieren mit je 500 ml Methylenchlorid werden die organischen Phasen vereinigt, über Natriumsulfat getrocknet und eingedampft. Man erhält 13,7 g der Titelverbindung als gelbes Harz, das ohne weitere Reinigung für die Cyclisierung eingesetzt wird.

b) [2-Methyl-spiro-(3-oxo-1-thiolan-5,4')]-1'-äthoxycarbonyl-piperidin

Das unter a erhaltene Harz wird in 120 ml Toluol aufgenommen, auf - 10 ° gekühlt und mit 40 ml Acetaldehyd versetzt. Nun werden bei gleichbleibender Temperatur langsam 15 ml Bortrifluorid-ätherat zugetropft. Man rührt noch 2 Stunden nach und arbeitet dann wie folgt auf:

Die rotbraune Suspension wird mit 150 ml 2 N Natronlauge-Lösung basisch gestellt und dreimal mit 300 ml Methylenchlorid extrahiert. Die vereinigten organischen Phasen werden mit Wasser gewaschen, über Natriumsulfat getrocknet, filtriert und eingedampft. Der rotbraune, ölige Rückstand wird am Vakuum destilliert. Man erhält 4,1 g gelbliches Oel. Sdp.: 128 - 135 °/0,07 mm Hg.

Beispiel 29: [2-Methyl-spiro-(3-oxo-1-thiolan-5,4')]-1'-methyl-piperidin

Diese Verbindung wird analog zu Beispiel 28 hergestellt. Hydrogenmaleinat aus Aethanol/Aether: Smp.: 139 - 142 °.

Beispiel 30: cis- und trans-[2-Methyl-spiro-(3-oxo-1-thiolan-5,3')]-1'-methyl-pyrrolidin

Das Isomerengemisch wird analog zu Beispiel 28 hergestellt und chromatographisch in die cis-Diastereomeren und die trans-Diastereomeren (jeweils Racemate) aufgetrennt:

| Beisp. 30a: cis-Diastereomeren | Beisp. 30b: trans-Diastereomeren |
|---|---|
| Hydrochlorid aus Methylenchlorid/Aether Smp.: 136 - 139 ° | Hydrochlorid aus Methylenchlorid/Aether Smp.: 165 - 168 ° |

Beispiel 31: [2-Methyl-spiro-(1,3-dioxolan-4,4')]-1'-methyl-piperidin

30 g Lithiumaluminiumhydrid werden in 1 Liter absolutem Tetrahydrofuran vorgelegt. Man tropft unter Stickstoff bei 0 ° während 2 Stunden 26 ml Schwefelsäure-monohydrat zu. Nach 15 Minuten Nachrühren wird während 30 Minuten unter gleichbleibender Temperatur eine Lösung von 18 g [2-Methyl-spiro-(1,3-dioxolan-4,4')]-1'-äthoxycarbonyl-piperidin (Herstellung siehe Beispiel 2) in 200 ml absolutem Tetrahydrofuran zugetropft.

Zur Aufarbeitung wird mit 1 Liter Aether verdünnt, mit 50 ml gesättigter Natriumsulfatlösung versetzt und während 30 Minuten gerührt. Die weisse Suspension wird über Hyflo filtriert, der Filterkuchen noch zweimal mit 500 ml Aether gewaschen und dann die vereinigten Filtrate eingedampft. Man erhält 10,2 g eines farblosen Oels, das als Hydrogenfumarat aus Aceton/Aether kristallisiert.

Smp.: 161 - 164 °.

Analog zu Beispiel 31 werden die Verbindungen der Beispiele 9, 11 bis 21, 23, 24, 26, 27, 29 und 30 hergestellt.

Beispiel 32: [2-Methyl-spiro-(1,3-dioxolan-4,4')]-1'-cyclopropylmethyl-piperidin

Eine Suspension von 5 g [2-Methyl-spiro-(1,3-dioxolan-4,4')]-piperidin (Verbindung des Beispiels 4), 5,1 g Cyclopropylmethylchlorid, 7,2 g Kaliumcarbonat und 4,5 g Kaliumjodid in 100 ml absolutem Dimethylformamid rührt man während 2 Stunden bei 80 °. Der Ansatz wird vollständig eingeengt und der feste Rückstand zwischen Wasser und Methylenchlorid verteilt. Die wässerige Phase wird noch zweimal mit Methylenchlorid extrahiert und die organischen Extrakte der Reihe nach mit wenig Wasser nachgewaschen, über Natriumsulfat getrocknet und zum orangen Oel eingeengt. Nach Chromatographie an der 20-fachen Menge Kieselgel mit Methylenchlorid/2 % Methanol als Elutionsmittel erhält man ein gelbliches Oel, das als Hydrogenmaleinat aus Aceton/Aether kristallisiert.

Smp. des Hydrogenmaleinats der Titelverbindung: 147 - 150 °.

Analog zu Beispiel 28 werden die Verbindungen der Beispiele 3, 5, 6, 9, 11 bis 21, 23, 24, 26, 27, 29 und 30 hergestellt.

Patentansprüche:

1. Eine Spiro-Verbindung, enthaltend einen fünfgliedrigen, carbocyclischen Ring, der in den Stellungen 1 und 3 durch zwei Heteroatome aus der Reihe Sauerstoff und Schwefel unterbrochen ist und dessen Kohlenstoffatom in Stellung 4 mit einem 4- bis 8-gliedrigen, carbocyclischen Ring, der durch ein Stickstoffatom unterbrochen ist, gemeinsam ist, in Form der freien Base oder eines pharmazeutisch annehmbaren Säureadditionssalzes oder quaternären Ammonium-Salzes, zur Anwendung als Therapeutikum.

2. Eine Verbindung der Formel I,

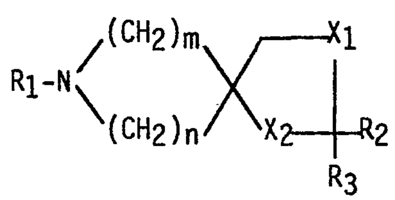

I

worin

$R_1$ für Wasserstoff, gegebenenfalls durch 1 bis 6 Halogenatome mit einer Atomzahl von 9 bis 35 substituiertes Alkyl mit 1 bis 6 Kohlenstoffatomen, Alkenyl oder Alkynyl mit je 3 bis 6 Kohlenstoffatomen, wobei die Mehrfachbindung nicht zu dem Stickstoffatom benachbart ist, Cycloalkyl mit 3 bis 7 Kohlenstoffatomen, Cycloalkyl(mit 3 bis 7 Kohlenstoffatomen)-alkyl(mit 1 oder 2 Kohlenstoffatomen), Benzyl, Alkoxycarbonyl mit 2 bis 5 Kohlenstoffatomen, Benzoxycarbonyl, Alkanoyl mit 2 bis 5 Kohlenstoffatomen, Benzoyl, Nicotinoyl, Dihydronicotinoyl oder N-Alkyl(mit 1 bis 4 Kohlenstoffatomen)-dihydronicotinoyl steht,

$R_2$ und $R_3$ unabhängig voneinander Wasserstoff, gegebenenfalls durch 1 bis 6 Halogenatome mit einer Atomzahl von 9 bis 35 substituiertes Alkyl mit 1 bis 6 Kohlenstoffatomen, Alkenyl oder Alkynyl mit je 2 bis 6 Kohlenstoffatomen, Cycloalkyl mit 3 bis 7 Kohlenstoffatomen oder Phenyl bedeuten oder zusammen eine $-(CH_2)_p$-Kette bilden, wobei p für 2, 3, 4 oder 5 steht,

$X_1$ und $X_2$ unabhängig voneinander Sauerstoff oder Schwefel bedeuten, und

m und n unabhängig voneinander für 1, 2, 3 oder 4 stehen, wobei m + n nicht mehr als 6 beträgt,

und ihre pharmazeutisch annehmbaren Säureadditionssalze und quaternären Ammonium-Salze,

zur Anwendung als Therapeutikum.

3. Eine Verbindung gemäss Anspruch 1 oder 2, zur Anwendung in der Behandlung der senilen Demenz, der Alzheimer'schen Krankheit, der Huntingtons Chorea, der tardiven Diskinesia, der Hyperkinesia, der akuten Verwirrtheitszuständen, der Manie und des Glaukoms.

4. Eine pharmazeutische Zusammensetzung, enthaltend als Wirkstoff eine Verbindung gemäss Anspruch 1 oder 2.

5. Eine pharmazeutische Zusammensetzung gemäss Anspruch 4, die zusätzlich einen peripheren cholinergen Blocker enthält.

6. Eine Verbindung gemäss Anspruch 1 mit Ausnahme von dem 3,7-Dimethyl-2,4-dioxo-7-azaspiro[3,4]octan und dessen Iodomethylat, dem [2,2-Dimethyl-spiro-(1,3-dioxolan-4,3')]-1'-methyl-piperidin-2-on, dem Spiro-(1,3-dioxolan-4,4')-1'-t.-butyl-piperidin und dem [2,2-Dimethyl-spiro-(1,3-dioxolan-4,4')]-1'-t.-butyl-piperidin.

7. Eine Verbindung gemäss Anspruch 2, worin $X_1$ und $X_2$ nicht beide Sauerstoff bedeuten, falls

a) $R_1$ und $R_2$ je Methyl bedeuten, $R_3$ für Wasserstoff steht und m und n beide für 1 stehen, oder

b) $R_1$ t.-Butyl bedeutet, $R_2$ und $R_3$ beide für Wasserstoff oder beide für Methyl und m und n beide für 2 stehen.

8. Eine Verbindung der Formel 7, als freie Base oder als Säureadditionssalz.

9. Das (-)-[2-Methyl-spiro-(1,3-dioxolan-4,4')]-1'-methyl-piperidin und seine pharmazeutisch annehmbaren Säureadditionssalze und quaternären Ammonium-Salze.

10. Verfahren zur Herstellung einer Spiro-Verbindung gemäss Anspruch 6, enthaltend die Stufe, dass man eine entsprechende 4- bis 8-gliedrige, durch ein Stickstoffatom unterbrochene, carbocyclische Verbindung, die anstelle des fünfgliedrigen heterocyclischen Ringes eine Gruppe aus der Reihe Hydroxy und Mercapto und eine Gruppe aus der Reihe Hydroxymethyl und Mercaptomethyl aufweist, cyclisiert und die erhaltene Spiro-Verbindung oder eine daraus hergestellte, weitere Spiro-Verbindung gemäss Anspruch 6, in Form der freien Base oder eines pharmazeutisch annehmbaren Säureadditionssalzes oder quaternären Ammonium-Salzes gewinnt.

11. Verfahren zur Herstellung einer Verbindung gemäss Anspruch 7, enthaltend die Stufe, dass man eine Verbindung der Formel II,

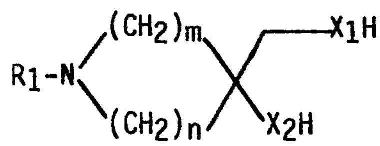

II

worin $R_1$, $X_1$, $X_2$, m und n wie im Anspruch 2 definiert sind, zu einer Verbindung der Formel I cyclisiert und die erhaltene Verbindung der Formel I oder eine daraus hergestellte, weitere Verbindung der Formel I, in Form der freien Base oder eines pharmazeutisch annehmbaren Säureadditionssalzes oder quaternären Ammonium-Salzes gewinnt.

Patentansprüche für AT

1. Verfahren zur Herstellung einer Spiro-Verbindung, enthaltend einen fünfgliedrigen, carbocyclischen Ring, der in den Stellungen 1 und 3 durch zwei Heteroatome aus der Reihe Sauerstoff und Schwefel unterbrochen ist und dessen Kohlenstoffatom in Stellung 4 mit einem 4- bis 8-gliedrigen, carbocyclischen Ring, der durch ein Stickstoffatom unterbrochen ist, gemeinsam ist, in Form der freien Base oder eines pharmazeutisch annehmbaren Säureadditionssalzes oder quaternären Ammonium-Salzes, enthaltend die Stufe, dass man eine entsprechende 4- bis 8-gliedrige, durch ein Stickstoffatom unterbrochene, carbocyclische Verbindung, die anstelle des fünfgliedrigen heterocyclischen Ringes eine Gruppe aus der Reihe Hydroxy und Mercapto und eine Gruppe aus der Reihe Hydroxymethyl und Mercaptomethyl aufweist, cyclisiert und die erhaltene Spiro-Verbindung oder eine daraus hergestellte, weitere oben definierte Spiro-Verbindung, in Form der freien Base oder eines pharmazeutisch annehmbaren Säureadditionssalzes oder quaternären Ammonium-Salzes gewinnt.

2. Verfahren zur Herstellung einer Verbindung der Formel I,

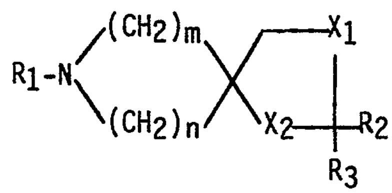

I

worin

$R_1$ für Wasserstoff, gegebenenfalls durch 1 bis 6 Halogenatome mit einer Atomzahl von 9 bis 35 substituiertes Alkyl mit 1 bis 6 Kohlenstoffatomen, Alkenyl oder

Alkynyl mit je 3 bis 6 Kohlenstoffatomen, wobei die Mehrfachbindung nicht zu dem Stickstoffatom benachbart ist, Cycloalkyl mit 3 bis 7 Kohlenstoffatomen, Cycloalkyl(mit 3 bis 7 Kohlenstoffatomen)-alkyl(mit 1 oder 2 Kohlenstoffatomen), Benzyl, Alkoxycarbonyl mit 2 bis 5 Kohlenstoffatomen, Benzoxycarbonyl, Alkanoyl mit 2 bis 5 Kohlenstoffatomen, Benzoyl, Nicotinoyl, Dihydronicotinoyl oder N-Alkyl(mit 1 bis 4 Kohlenstoffatomen)-dihydronicotinoyl steht,

$R_2$ und $R_3$ unabhängig voneinander Wasserstoff, gegebenenfalls durch 1 bis 6 Halogenatome mit einer Atomzahl von 9 bis 35 substituiertes Alkyl mit 1 bis 6 Kohlenstoffatomen, Alkenyl oder Alkynyl mit je 2 bis 6 Kohlenstoffatomen, Cycloalkyl mit 3 bis 7 Kohlenstoffatomen oder Phenyl bedeuten oder zusammen eine $-(CH_2)_p$-Kette bilden, wobei p für 2, 3, 4 oder 5 steht,

$X_1$ und $X_2$ unabhängig voneinander Sauerstoff oder Schwefel bedeuten, und

m und n unabhängig voneinander für 1, 2, 3 oder 4 stehen, wobei m + n nicht mehr als 6 beträgt,

in Form der freien Base oder eines pharmazeutisch annehmbaren Säureadditionssalzes oder quaternären Ammonium-Salzes, enthaltend die Stufe, dass man eine Verbindung der Formel II,

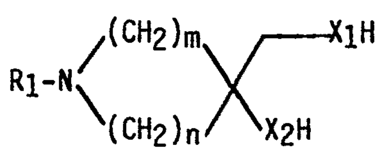

II

worin $R_1$, $X_1$, $X_2$, m und n obige Bedeutung besitzen, zu einer Verbindung der Formel I cyclisiert und die erhaltene Verbindung der Formel I oder eine daraus hergestellte, weitere Verbindung der Formel I, in Form der freien Base oder eines pharmazeutisch annehmbaren Säureadditionssalzes oder quaternären Ammonium-Salzes gewinnt.

3. Verfahren gemäss Anspruch 2, dadurch gekennzeichnet, dass man die Verbindung der Formel II mit einer Verbindung der Formel III,

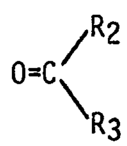

III

worin $R_2$ und $R_3$ wie im Anspruch 2 definiert sind, umsetzt.

4. Verfahren gemäss Anspruch 3, zur Herstellung von Verbindungen der Formel I in Form der freien Basen oder von Säureadditionssalzen.

5. Verfahren gemäss Anspruch 2 oder 3, zur Herstellung des (-)-[2-Methyl-spiro-(1,3-dioxolan-4,4')]-1'-methyl-piperidins und seiner pharmazeutisch annehmbaren Säureadditionssalze und quaternären Ammonium-Salze.

6. Eine Spiro-Verbindung, enthaltend einen fünfgliedrigen, carbocyclischen Ring, der in den Stellungen 1 und 3 durch zwei Heteroatome aus der Reihe Sauerstoff und Schwefel unterbrochen ist und dessen Kohlenstoffatom in Stellung 4 mit einem 4- bis 8-gliedrigen, carbocyclischen Ring, der durch ein Stickstoffatom unterbrochen ist, gemeinsam ist, in Form der freien Base oder eines pharmazeutisch annehmbaren Säureadditionssalzes oder quaternären Ammonium-Salzes, hergestellt nach dem Verfahren gemäss Anspruch 1.

7. Eine Verbindung der Formel I gemäss Anspruch 2, hergestellt nach dem Verfahren gemäss einem der Ansprüche 2 bis 5.

8. Eine pharmazeutische Zusammensetzung, enthaltend als Wirkstoff eine Verbindung gemäss Anspruch 6 oder 7.

9. Verfahren zur Herstellung einer pharmazeutischen Zusammensetzung gemäss Anspruch 8, dadurch gekennzeichnet, dass man eine Verbindung gemäss Anspruch 6 oder 7 mit einem pharmakologisch indifferenten Hilfs- oder Trägerstoff vermischt.